# EUROPEAN PATENT SPECIFICATION

(11) **EP 3 270 693 B1**
(45) Date of publication and mention of the grant of the patent: **30.06.2021**
(21) Application number: 16765810.3
(22) Date of filing: 18.03.2016
(51) Int. Cl.: A01N 37/00, A61K 31/20, A61P 9/10

(54) **METHODS FOR STABILIZING ATHEROSCLEROTIC PLAQUES USING LIPOXINS, RESOLVINS, AND ANALOGS THEREOF**
VERFAHREN ZUR STABILISIERUNG VON ATHEROSKLEROTISCHEN PLAQUES UNTER VERWENDUNG VON LIPOXINEN, RESOLEN UND ANALOGA DAVON
PROCÉDÉS POUR STABILISER DES PLAQUES D'ATHÉROSCLÉROSE À L'AIDE DE LIPOXINES, DE RÉSOLVINES ET DE LEURS ANALOGUES

(30) Priority: 18.03.2015 US 201562134894 P
(43) Date of publication of application: 24.01.2018
(73) Proprietor: Forsyth Dental Infirmary for Children, Cambridge, MA 02142 (US)
(72) Inventor: VAN DYKE, Thomas, E., West Roxbury, MA 02132 (US); HASTURK, Hatice, Brighton, MA 02135 (US); KANTARCI, Alpdogan, Brighton, MA 02135 (US); HAMILTON, James, A., Newton, MA 02159 (US)
(74) Representative: Lock, Graham James
(86) International application number: PCT/US2016/023067
(87) International publication number: WO 2016/149582

(56) References cited:
- WO-A1-2008/068041
- WO-A1-2010/039531
- WO-A2-2004/014835
- WO-A2-2014/039964
- US-A- 5 441 951
- US-A- 5 650 435
- US-A1- 2014 079 631
- FREDMAN ET AL.: 'Targeted nanoparticles containing the proresolving peptide Ac2-26 protect against advanced atherosclerosis in hypercholesterolemic mice' SCI TRANSL MED. vol. 7, no. 275, 18 February 2015, page 275RA20, XP055312681

## Description

### Background of the Invention

Atherosclerosis is a chronic, lipoprotein-driven inflammatory disease that causes luminal narrowing of the arteries due to the development of lesions, or atherosclerotic plaques (Ross (1999) N Engl. J. Med. 340:115-126). The plaques that develop as a result of the disease are often separated into two categories - stable and unstable plaques - with each category having distinct biological and morphological properties. Stable atherosclerotic plaques are characterized by large amounts of extracellular matrix (ECM) and smooth muscle cells, while unstable, or so-called vulnerable plaques, are comprised predominantly of macrophages and foam cells, covered by minimal amounts of ECM. When this thin layer of ECM, often referred to as the fibrous cap, ruptures, a thrombus (i.e. blood clot) forms within the lumen of the artery and can cause total occlusion of the artery or occlusion elsewhere in the downstream vasculature (Finn et al. (2010) Arterioscler. Thromb. Vasc. Biol. 30(7): 1282-92; Bentzon et al. (2014) Circ. Res. 6:1852-1866). The development and rupture of such plaques may suddenly cause life-threatening coronary thrombosis presenting as an acute myocardial infarction or heart attack. Atherosclerotic plaques can often lead to the precipitation of thrombi that obstruct blood flow to the heart, brain, or lower extremities, resulting in coronary heart disease, ischemic stroke, or peripheral vascular disease, respectively.

The mechanisms involved in plaque erosion remain largely unknown, though there is evidence from animal studies and human genetic studies that the pro-inflammatory pathways involving leukotrienes and lipoxygenases (the enzymes necessary for leukotriene synthesis) play a role in atherosclerosis, and possibly in the development of vulnerable plaques (Dwyer et al. (2004) N. Engl. J. Med. 350:29-37; Zhou et al. (2010) Cardiology 115:221-228). In biological processes where inflammation is present, the early actions of the host inflammatory response are later replaced by more specific inflammation-resolving mechanisms. Little is known about the effect of the anti-inflammatory lipid mediators, such as lipoxins and resolvins, in atherosclerosis. A recent paradigm in periodontal disease pathogenesis emphasizes the importance of these counterregulatory molecules ("off signals") in the resolution of inflammatory response to control its magnitude and duration (Van Dyke (2011) J. Clin. Periodontol. 38 (Suppl. 11) 119:125). Omega-3 and omega-6 polyunsaturated fatty acids (n-3 and n-6 PUFA) are essential fatty acids provided by dietary sources and exert anti-inflammatory effects to limit the inflammatory cascade due to their hypolipidemic properties. Resolvins are derived from omega-3 fatty acids, EPA and DHA (resolvin E1 and resolvin D1 respectively), while lipoxins are derived from the omega-6 fatty acid arachidonic acid (AA). The anti-inflammatory and proresolving actions of the resolvins and protectins (also derived from DHA) have already been documented in several animal models of inflammatory diseases and tissue injury including periodontal disease (Serhan et al. (2003) J. Immunol. 171:6856-6865; Hasturk et al. (2006) FASEB J. 20:401-403; Hasturk et al. (2007) J. Immunol. 179:7021-7029).

Despite these results, however, few agonists of endogenous resolution programs, as opposed to inhibitors of the inflammatory process or immunosuppressives, are known. Rather than treating or preventing the root causes of arterial conditions that would benefit from increased plaque stabilization, current practices are largely limited to surgical procedures (e.g. stenting) to treat the results. Prevention strategies and associated therapies are generally aimed at lowering cholesterol levels (i.e. the use of statins). Thus, there is a great need to identify agents that can increase the stabilization of vulnerable atherosclerotic plaques and methods for treating and preventing arterial conditions that would benefit from increased plaque stabilization using such agents.

WO 2010/039531 describes certain resolvin compounds for inhibiting collagen-induced arthritis and delayed-type-hypersensitivity (DTH) in mouse models.

WO 2004/014835 discloses certain resolvin compounds for reducing of neutrophil infiltration in Zymosan-induced peritonitis.

WO 2008/068041 discloses synthesis and preparation of hydrate forms of Lipoxin A₄.

US 5441951 and US 5650435 disclose certain lipoxin compounds for inhibiting neutrophil adhesion to endothelial cells and their transmigration on epithelial cells.

### Summary of the Invention

The present invention is based, at least in part, on the discovery that lipoxin A₄ (LXA₄), lipoxin analogs, members of the resolvin class of compounds (e.g. Resolvin D1 and Resolvin E1), and analogs thereof can stabilize atherosclerotic plaques. Thus, in one aspect, the present invention provides a composition comprising a therapeutically effective amount of 9,12 benzo-LXA4 or Resolvin E1 for use in stabilizing an atherosclerotic plaque and/or reducing unstable/vulnerable plaque in a subject.

In one embodiment, the 9,12 benzo-LXA₄ or Resolvin E1 can be formulated in a pharmaceutically acceptable carrier, such as in mouth rinses, chewing gum, food additives, lozenges, tablet, chewable capsule, intraoral delivery devices, or encapsulated in resporbable carrier nanoparticles of biologic or synthetic origin. In still another embodiment, the effective amount of the 9,12 benzo-LXA₄ or Resolvin E1 is an amount that increases stabilization of a vulnerable plaque. In yet another embodiment, the method further comprises administering at least one additional agent selected from the group consisting of anti-inflammatory agents.

In another aspect, the present invention provides a composition comprising therapeutically effecting amount of 9,12 benzo-LXA4 or Resolving E1, for use in stabilizing an atherosclerotic plaque and/or reducing unstable/vulnerable plaque in a subject in need thereof comprising the steps of: a) performing surgery upon at least one arterial lesion of the subject; and b) administering the composition to the surgical wound at the time of surgery; to thereby stabilize an atherosclerotic plaque and/or reduce unstable/vulnerable plaque in the subject. In one embodiment, 9,12 benzo-LXA₄ or Resolvin E1 is formulated in a pharmaceutically acceptable carrier (nanoparticle of biologic or synthetic origin). In still another embodiment, the pharmaceutically acceptable carrier is selected from the group consisting of mouth rinses, chewing gum, food additives, lozenges, tablet, chewable capsule, and intraoral delivery devices. In yet another embodiment, the effective amount of the 9,12 benzo-LXA₄ or Resolvin E1 is an amount that increases plaque stability. In another embodiment, the method further comprises administering at least one additional agentselected from the group consisting of anti-inflammatory agents. In yet another embodiment, the administration is topical.

In addition, the detailed description provided herein discloses additional aspects of the present invention.

### Brief Description of Figures

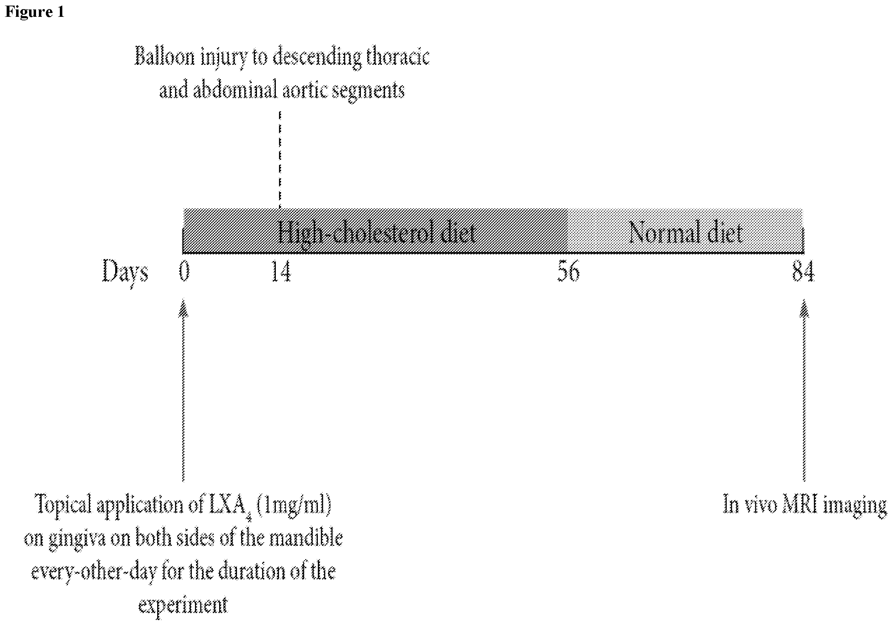

**Figure 1** shows a schematic diagram of an experimental protocol for treatment of vulnerable atherosclerotic plaques with Resolvin E1.

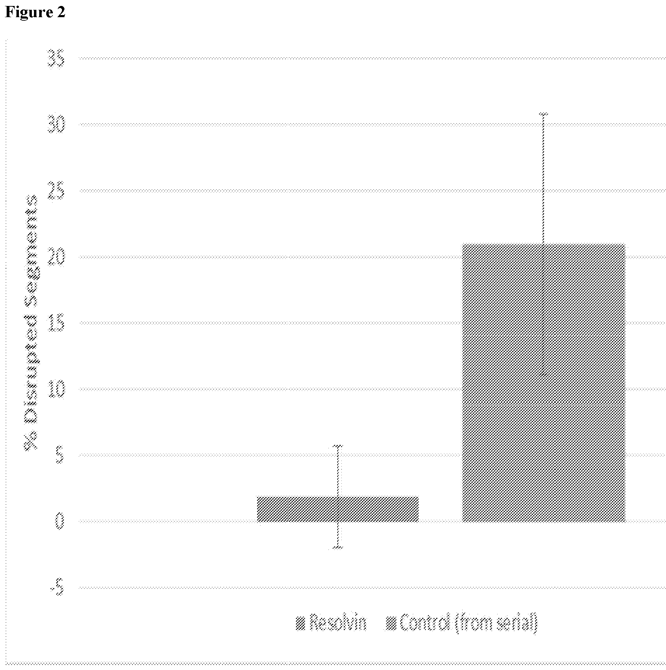

**Figure 2** shows the percentage of disrupted plaques per rabbit for the ResolvinE1-treated and control group in the atherosclerosis animal model study.

### Detailed Description of the Invention

The present invention is based, at least in part, on the discovery that 9,12-LXA₄ and Resolvin E1 can increase plaque stability and restore vulnerable plaques to a more stable phenotype in a rabbit model of atherosclerosis. Application of 9,12-LXA4 or Resolvin E1 to both sides of the mandible on the gingiva every-other-day was unexpectedly sufficient to stabilize atherosclerotic plaques that had developed as a result of a high cholesterol diet and balloon injury to the descending thoracic and abdominal aortic segments. Accordingly, a composition comprising a therapeutically effective amount of 9,12 benzp-LXA₄ or Resolvin E1 for use in stabilizing an atherosclerotic plaque and/or reducing stability unstable/vulnerable plaque in a subject is provided herein. Methods for monitoring the progress of treating such disorders, as well as predicting the response to such treatment, are described herein.

### A. Definitions

In order that the present invention may be more readily understood, certain terms are first defined. Additional definitions are set forth throughout the detailed description.

The articles "a" and "an" are used herein to refer to one or to more than one (i.e. to at least one) of the grammatical object of the article. By way of example, "an element" means one element or more than one element.

The term "analog" refers to any molecule having the basic structural components of a parent compound. Analogs of lipoxins and resolvins encompass compounds containing a carboxyl component, a diol component, a tetraene component, and an alcohol component. These components can be any size and can be joined to one another in any manner. Additionally, these components can contain various substituents or have some of their carbon atoms replaced, for example, by rings or heteroatoms. Such analogs also retain at least one lipoxin activity but do not undergo the typical metabolic deactivation of the parent lipoxin compounds such that the in vivo half life of the compounds it significantly greater than that of the parent compounds. In some embodiments, lipoxin analogs have an "active region" that functions like the active region of a "natural lipoxin," but which has a "metabolic transformation region" that differs from the natural lipoxin. Such lipoxin analogs include compounds which are structurally similar to a natural lipoxin, compounds which share the same receptor recognition site, compounds which share the same or similar lipoxin metabolic transformation region as lipoxin, and compounds which are art-recognized as being analogs of lipoxin. Lipoxin analog metabolites are also included. The compounds disclosed herein may contain one or more centers of asymmetry. Where asymmetric carbon atoms are present, more than one stereoisomer is possible, and all possible isomeric forms are intended to be included within the structural representations shown. Optically active (R) and (S) isomers may be resolved using conventional techniques known to the ordinarily skilled artisan. The present invention is intended to include the possible diastereiomers as well as the racemic and optically resolved isomers. In this context, the term "corresponding lipoxin" and "natural lipoxin" refer to a naturally-occurring lipoxin or lipoxin metabolite. An "active region" refers to the region of a natural lipoxin or lipoxin analog, which is associated with in vivo cellular interactions. The active region may bind the "recognition site" of a cellular lipoxin receptor or a macromolecule or complex of macromolecules, including an enzyme and its cofactor. Lipoxin A₄ analogs have an active region comprising C5-C15 of natural lipoxin A₄. The term "metabolic transformation region" is intended to refer generally to that portion of a lipoxin, a lipoxin metabolite, or lipoxin analog including a lipoxin analog metabolite, upon which an enzyme or an enzyme and its cofactor attempts to perform one or more metabolic transformations which that enzyme or enzyme and cofactor normally transform on lipoxins. The metabolic transformation region may or may not be susceptible to the transformation. A non-limiting example of a metabolic transformation region of a lipoxin is a portion of LXA₄ that includes the C-13,14 double bond or the C-15 hydroxyl group, or both. The term "inhibits metabolism" means the blocking or reduction of activity of an enzyme which metabolizes a native lipoxin. The blockage or reduction may occur by covalent bonding, by irreversible binding, by reversible binding which has a practical effect of irreversible binding, or by any other means which prevents the enzyme from operating in its usual manner on another lipoxin analog, including a lipoxin analog metabolite, a lipoxin, or a lipoxin metabolite. The term "resists metabolism" is meant to include failing to undergo one or more of the metabolic derivative transformations by at least one of the enzymes which metabolize lipoxins. Two non-limiting examples of LXA₄ analog that resists metabolism are 1) a structure which can not be oxidized to the 15-oxo form, and 2) a structure which may be oxidized to the 15-oxo form, but is not susceptible to enzymatic reduction to the 13,14-dihydro form. The term "more slowly undergoes metabolism" means having slower reaction kinetics, or requiring more time for the completion of the series of metabolic transformations by one or more of the enzymes which metabolize lipoxin. A non-limiting example of an LXA₄ analog which more slowly undergoes metabolism is a structure which has a higher transition state energy for C-15 dehydrogenation than does LXA₄ because the analog is steric ally hindered at the C-16.

The term "binding" or "interacting" refers to an association, which may be a stable association, between two molecules, *e.g.*, between a polypeptide of the invention and a binding partner, due to, for example, electrostatic, hydrophobic, ionic and/or hydrogen-bond interactions under physiological conditions. Exemplary interactions include protein-protein, protein-nucleic acid, protein-small molecule, and small molecule-nucleic acid interactions.

The term "biological sample" when used in reference to a diagnostic assay is intended to include tissues, cells and biological fluids isolated from a subject, as well as tissues, cells and fluids present within a subject.

The term "body fluid" refers to fluids that are excreted or secreted from the body as well as fluids that are normally not (*e.g.* amniotic fluid, aqueous humor, bile, blood and blood plasma, cerebrospinal fluid, ceriman and earwax, Cowper's fluid or pre-ejaculatory fluid, chili, chime, stool, female ejaculate, interstitial fluid, intracellular fluid, lymph, menses, breast milk, mucus, pleural fluid, pus, saliva, sebum, semen, serum, sweat, synovial fluid, tears, urine, vaginal lubrication, vitreous humor, vomit).

### B. Subjects

The terms "subject" and "patient" are used interchangeably. As used herein, the terms "subject" and "subjects" refer to an animal, *e.g.,* a mammal including a non-primate (*e.g.,* a cow, pig, horse, donkey, goat, camel, cat, dog, guinea pig, rat, mouse, sheep) and a primate (*e.g.,* a monkey, such as a cynomolgous monkey, gorilla, chimpanzee and a human). In one embodiment, the subject for whom treatment or prevention of an arterial disorder that would benefit from increased plaque stabilization is a mammal (*e.g.*, mouse, rat, primate, non-human mammal, domestic animal such as dog, cat, cow, horse), and is preferably a human. In some embodiments, the human is afflicted with or is suspected of having an arterial disorder that would benefit from increased plaque stabilization.

The terms "arterial disorder" and "arterial disorder that would benefit from increased plaque stabilization" includes a disorder, disease or condition which is caused or characterized by arterial plaques with an abnormally high composition of macrophages and foam cells.

### C. LXA4, LXA4 analogs, and oral formulations thereof

Lipoxins are naturally-occurring lipid mediators derived from the fatty acid, arachidonic acid (Bazan (2006) in Basic Neurochemistry: Molecular, Cellular and Medical Aspects, 7th edition, G. Siegel et al. (eds.), Chapter 33:575-591; Mattson and Bazan (2006) in Basic Neurochemistry: Molecular, Cellular and Medical Aspects, 7th edition, G. Siegel et al. (eds.), Chapter 35:603-615. Lipoxins are potent mediators of the resolution phase of the inflammatory response and of dysfunctional immunity (Serhan et al. (1999) Adv. Exp. Med. Biol. 469:287-293; Fiorucci et al. (2004) Proc. Natl. Acad. Sci. USA. 101:15736-15741). There are several classes of lipoxins, such as LXA₄ and LXB₄, as well as analogs thereof that have been discovered/synthesized since the initial discovery of lipoxins in the 1980s. Specifically, lipoxin A₄ and its analogs, including lipoxin A₄ epimer 15 (or 15-epi-lipoxin A₄), are well known in the art (U.S. Pat. Nos. 6,831,186; 6,645,978; and 8,093,417; U.S. Pat. Publ. 2012/0149771; Fierro et al. (2003) J. Immunol. 170:2688-2694; Bannenberg et al. (2004) Brit. J. Pharma. 143:43-52; and Scalia et al. (1997) Proc. Natl. Acad. Sci. USA 94:9967-9972).

LXA₄ analogs are also well known in the art. Benzo-lipoxins have been found to be thermally and metabolically more stable than either of the endogenous lipoxins (LXA₄ and LXB₄). Replacement of the tetraene unit of LXA₄ with a benzo-fused ring also allows for efficient synthesis of these analogs. 9,12-LXA₄ is a member of this class of benzo-lipoxins and has been shown to have potent anti-inflammatory properties in a mouse model of acute inflammation, significantly reducing polymorphonuclear leukocyte (PMN) infiltration and levels of pro-inflammatory cytokines in vivo (Sun et al. (2009) Prost. Leuokt. Essent. Fatty Acids 81:357-366; Petasis et al. (2008) Bioorg. Med. Chem. Lett. 18:1382-1387).

In some embodiments, LXA₄ analogs can have one of the following structures:

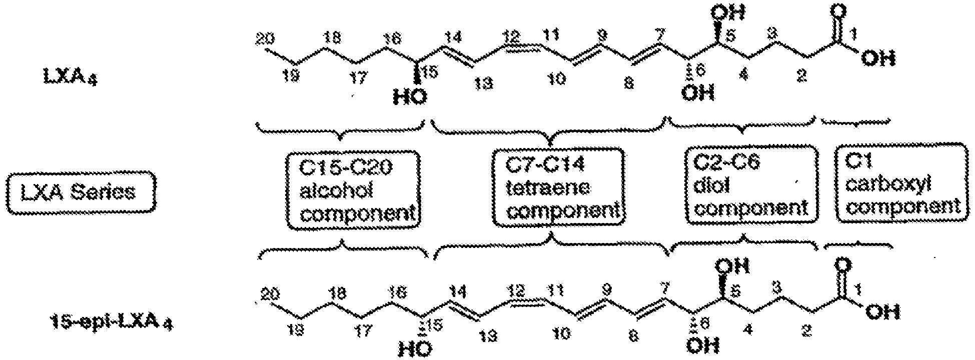

These can be expanded to include additional LXA₄ analogs having one of the following structures having the designated stereochemistry:

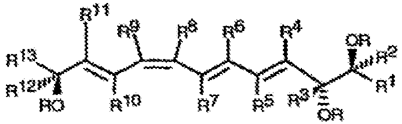

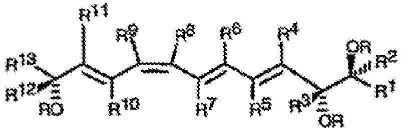

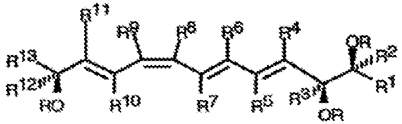

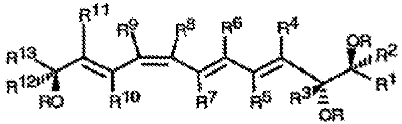

In these structures, the R-groups are independently selected as follows:
R is hydrogen or a straight, branched, cyclic, saturated, or unsaturated alkyl;
R¹, R², R¹², R¹³ are each independently selected from hydrogen; straight, branched, cyclic, saturated, or unsaturated alkyl having from 1 to 20 carbon atoms; substituted alkyl having from 1 to 20 carbon atoms, wherein the alkyl is substituted with one or more substituents selected from halo, hydroxy, lower alkoxy, aryloxy, amino, alkylamino, dialkylamino, acylamino, arylamino, hydroxyamino, alkoxyamino, alkylthio, arylthio, carboxy, carboxamido, carboalkoxy, aryl, and heteroaryl; substituted aryl or heteroaryl wherein the aryl or heteroaryl is substituted with one or more substituent selected from alkyl, cycloalkyl, alkoxy, halo, aryl, heteroaryl, carboxyl, and carboxamido; and a group Z-Y, wherein Z is a straight, branched, cyclic, saturated, or unsaturated alkyl having from 1 to 20 carbon atoms; substituted lower alkyl wherein the alkyl is substituted with one or more substituents selected from halo, hydroxy, lower alkoxy, aryloxy, amino, alkylamino, dialkylamino, acylamino, arylamino, hydroxyamino, alkoxyamino, alkylthio, arylthio, carboxy, carboxamido, carboalkoxy, aryl, and heteroaryl; substituted aryl or heteroaryl wherein the aryl or heteroaryl is substituted with one or more substituents selected from alkyl, cycloalkyl, alkoxy, halo, aryl, heteroaryl, carboxyl, and carboxamido; and Y is selected from hydrogen; alkyl; cycloalkyl; carboxyl; carboxamido; aryl; heteroaryl; substituted aryl or heteroaryl wherein the aryl or heteroaryl is substituted with one or more substituents selected from alkyl, cycloalkyl, alkoxy, halo, aryl, heteroaryl, carboxyl, and carboxamido;
R³ is selected from hydrogen; straight, branched, cyclic, saturated, or unsaturated alkyl having from 1 to 20 carbon atoms; substituted alkyl having from 1 to 20 carbon atoms, wherein the alkyl is substituted with one or more substituents selected from the group consisting of halo, hydroxy, lower alkoxy, aryloxy, amino, alkylamino, dialkylamino, acylamino, arylamino, hydroxyamino, alkoxyamino, alkylthio, arylthio, carboxy, carboxamido, carboalkoxy, aryl, and heteroaryl; substituted aryl or heteroaryl, wherein the aryl or heteroaryl is substituted with one or more substituents selected from the group consisting of alkyl, cycloalkyl, alkoxy, halo, aryl, heteroaryl, carboxyl, and carboxamido; and
R⁴-R¹¹ are selected from a group consisting of: hydrogen; halo; straight, branched, cyclic, saturated, or unsaturated alkyl having from 1 to 20 carbon atoms; substituted alkyl having from 1 to 20 carbon atoms, wherein the alkyl is substituted with one or more substituents selected from halo, hydroxy, lower alkoxy, aryloxy, amino, alkylamino, dialkylamino, acylamino, arylamino, hydroxyamino, alkoxyamino, alkylthio, arylthio, carboxy, carboxamido, carboalkoxy, aryl, and heteroaryl; substituted aryl or heteroaryl wherein the aryl or heteroaryl are substituted with one or more substituent selected from alkyl, cycloalkyl, alkoxy, halo, aryl, heteroaryl, carboxyl, and carboxamido;
R, R¹-R¹³ may be also connected to form one or more rings containing 3 to 20 carbon atoms, 1 to 6 oxygen atoms or 1 to 6 nitrogen atoms.

A pair selected among the R¹-R¹³ groups may also be replaced with a bond that generates a carbon-carbon double or triple bond or a ring.

Examples of exemplary, representative LXA₄ analogs are shown in Scheme 1. These examples are provided for purposes of illustration and in no way limit the scope of the present invention. Also contemplated as preferred compounds are the compounds shown in Scheme 1 wherein the carbon chains and rings shown in the structures additionally possess substituents selected from halo, hydroxy, lower alkoxy, aryloxy, amino, alkylamino, dialkylamino, acylamino, arylamino, hydroxyamino, alkoxyamino, alkylthio, arylthio, carboxy, carboxamido, carboalkoxy, aryl, and heteroaryl.

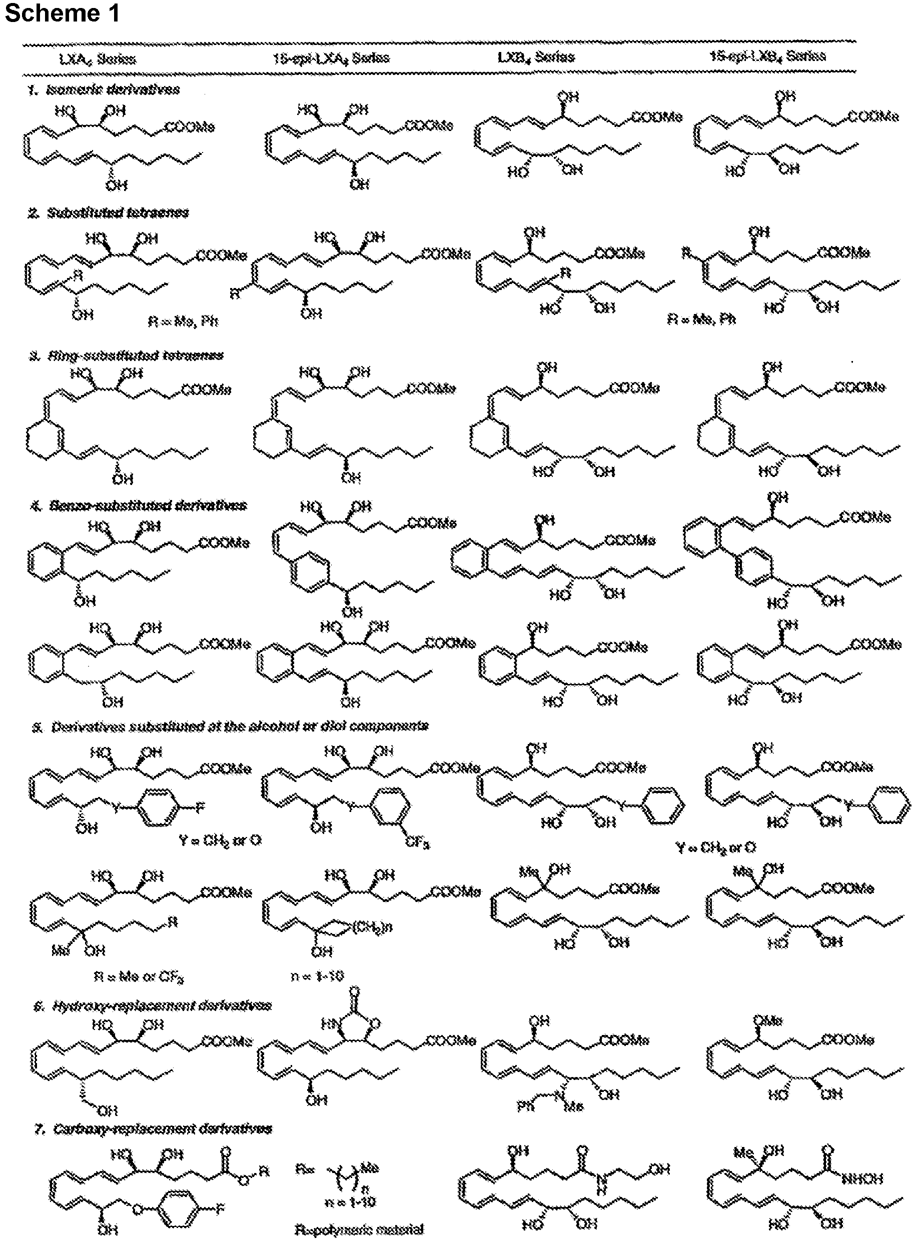

In some embodiments, LXA₄ and/or its analogs can be formulated with a physiologically compatible carrier medium. Such media can be of any simple type, *e.g.,* a pharmaceutically acceptable carrier such as fructo-oligo-saccharide (FOS) medium, or other soluble fiber, sugar, nutrient or base material for the composition, with which the LXA₄ and/or its analogs can be formulated, e.g., in an orally administrable form. Other non-limiting, exemplary carrier media include mannitol, inulin (a polysaccharide), polydextrose, arabinogalactan, polyolslactulose, lactitol, etc. A wide variety of materials can be used as carrier material in the practice of the present disclosure, as will be apparent to those of ordinary skill in the art, based on the description herein.

The carrier medium, when present, can be blended with LXA₄ and/or its analogs in any suitable amounts, such as an amount of from 5% to 95% by weight of carrier medium, based on the total volume or weight of LXA₄ and/or its analogs and the carrier medium. In some embodiments, the amount of carrier medium can be in a range having a lower limit of any of 5%, 10%, 12%, 15%, 20%, 25%, 28%, 30%, 40%, 50%, 60%, 70% or 75%, and an upper limit, higher than the lower limit, of any of 20%, 22%, 25%, 28%, 30%, 40%, 50%, 60%, 70%, 75%, 80%, 85%, 90%, and 95%. The amount of carrier medium in a specific embodiment may be determined based on considerations of the specific dose form, relative amounts of LXA₄ and/or its analogs, the total weight of the composition including the carrier medium and the bacterial species, and the physical and chemical properties of the carrier medium, and other factors, as known to those of ordinary skill in the LXA₄ formulation art.

LXA₄ and/or its analogs described herein can be formulated for oral administration. In some embodiments, the oral administration is targeted for application to the oral cavity, such as by applying the compositions and active ingredients contained therein to surfaces of the oral cavity, including but not limited to salivary glands, saliva, gingiva, teeth, tongue, cheek tissue, and the like. The term "orally acceptable carrier" refers to one or more safe solid or liquid diluents or encapsulating substances compatible with LXA₄ and/or its analogs described herein and are suitable for topical oral administration. The term "compatible" means the substance is capable of being mixed with the LXA₄ and/or its analogs without interaction in a manner which would substantially reduce the agent's stability and/or efficacy. Non-exclusive examples of such orally acceptable carriers include distilled or deionized water, calcium carbonate, calcium citrate, bentonite, and montmorillonite.

The term "oral care composition" refers to any composition suitable for administration to the oral cavity of a human or animal subject for enhancing the health of the subject, preferably providing such benefits as: the prevention or treatment of an arterial disorder that would benefit from increased plaque stabilization. The oral composition of the present invention may be in the form of a capsule, cachets, pills, lozenge, granules, subgingival gel, dentifrice, mouth rinse, mouth spray, oral tablet, oral device, chewing gum, oil-in-water emulsion, water-in-oil emulsion, elixir, syrup, or pastille using an inert base, such as gelatin and glycerin, or sucrose and acacia), or encapsulated in resporbable carrier nanoparticles of biologic or synthetic origin. Particles containing at least one component of a cellular-derived microparticle are described in L. Norling et al., Journal of Immunology, Cutting Edge: Humanized Nano-Proresolving Medicines Mimic Inflammation - Resolution and Enhance Wound Healing (published online April 1, 2011) and in WO2012/135032. The oral composition may also be incorporated onto strips or films for the application or attachment to oral surfaces.

In solid dosage forms for oral administration (capsules, tablets, pills, dragees, powders, granules and the like), the active ingredient is mixed with one or more pharmaceutically-acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, acetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered peptide or peptidomimetic moistened with an inert liquid diluent.

Tablets, and other solid dosage forms, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions, which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions, which can be used include polymeric substances and waxes. The active ingredient can also be in micro-encapsulated form, if appropriate, with one or more of the above-described excipients.

Liquid dosage forms for oral administration include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Suspensions, in addition to the active agent, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

In some embodiments, nanoparticle delivery vehicles can be used for oral administration.

The oral compositions for use in the present invention can be formulated with pharmaceutically acceptable carriers and/or diluents. The term "pharmaceutically acceptable" is employed herein to refer to those agents, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The term "pharmaceutically-acceptable carrier" as used herein means a pharmaceutically-acceptable material, composition or vehicle, such as a liquid or solid filler, diluent, excipient, solvent or encapsulating material, involved in carrying or transporting the subject chemical from one organ, or portion of the body, to another organ, or portion of the body. Each carrier must be "acceptable" in the sense of being compatible with the other ingredients of the formulation and not injurious to the subject. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

The term "pharmaceutically-acceptable salts" refers to the relatively non-toxic, inorganic and organic acid addition salts of the agents that modulates (*e.g.*, enhances) Fndc5 expression and/or activity, or expression and/or activity of the complex encompassed by the invention. These salts can be prepared in situ during the final isolation and purification of the respiration uncoupling agents, or by separately reacting a purified respiration uncoupling agent in its free base form with a suitable organic or inorganic acid, and isolating the salt thus formed. Representative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts and the like (See, for example, Berge et al. (1977) "Pharmaceutical Salts", J. Pharm. Sci. 66:1-19).

In addition, the oral compositions useful for the present invention can comprise ingredients that are useful for more than simple oral formulation binding. Such optional ingredients generally are used individually at levels from about 0.0005% to about 10.0%, preferably from about 0.005% to about 1.0% by weight of the composition.

Examples of suitable optional ingredients include, fluoride ion sources, alkali metal bicarbonate sources, humectants, anticalculus agents, abrasive polishing materials, thickening materials, surfactants, titanium dioxide, flavoring and sweetening agents, xylitol, coloring agents, teeth whitening agents, bentonite, montmorillonite, other active ingredients, and mixtures thereof.

Examples of suitable fluoride ion sources include, sodium fluoride, potassium fluoride, sodium monofluorophosphate.

Examples of suitable alkali metal bicarbonate sources include, sodium bicarbonate, and potassium bicarbonate.

Examples of suitable humectants include, water soluble liquid polyols selected from the group comprising glycerine, propylene glycol, sorbitol, xylitol, butylene glycol, polyethylene glycol, and mixtures thereof.

Examples of suitable thickening materials include, carboxyvinyl polymers, carrageenan, hydroxyethyl cellulose, water soluble salts of cellulose ethers such as sodium carboxymethylcellulose and sodium carboxymethyl hydroxyethyl cellulose, natural gums such as gum karaya, xanthan gum, gum arabic, and gum tragacanth, and mixtures thereof.

Examples of suitable surfactants include, sodium and potassium salts of the following: lauroyl sarcosinate, myristoyl sarcosinate, palmitoyl sarcosinate, stearoyl sarcosinate and oleoyl sarcosinate.

Examples of suitable flavoring agents include, oil of wintergreen, oil of peppermint, oil of spearmint, clove bud oil, menthol, anethole, methyl salicylate, eucalyptol, cassia, 1-menthyl acetate, sage, eugenol, parsley oil, oxanone, alpha-irisone, marjoram, lemon, orange, propenyl guaethol, cinnamon, vanillin, thymol, linalool, cinnamaldehyde glycerol acetal known as CGA, and mixtures thereof.

Examples of suitable sweetening agents include, sucrose, glucose, saccharin, dextrose, levulose, lactose, mannitol, sorbitol, fructose, maltose, xylitol, saccharin salts, thaumatin, aspartame, D-tryptophan, dihydrochalcones, acesulfame and cyclamate salts, especially sodium cyclamate and sodium saccharin, and mixtures thereof.

Examples of suitable antimicrobial agents include, phenol and its homologs, mono and poly-alkyl and aromatic halophenols, resorcinol and its derivatives, bisphenolic compounds and halogenated salicylanilides, benzoic esters, and halogenated phenols, quaternary ammonium agents, copper bisglycinate, copper glycinate, zinc citrate, zinc lactate, chlorhexidine, triclosan, triclosan monophosphate, and flavor oils such as thymol.

Examples of suitable enzymes include, proteases including papain, pepsin, trypsin, ficin, bromelin; cell wall lytic enzymes including lysozyme; plaque matrix inhibitors including dextranases, mutanases; and oxidases including glucose oxidase, lactate oxidase, galactose oxidase, uric acid oxidase, peroxidases including horse radish peroxidase, myeloperoxidase, lactoperoxidase, and chloroperoxidase.

Examples of antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

In some embodiments, the oral compositions can further comprise one or more agents or regimens that can further treat the desired arterial disorder that would benefit from increased plaque stabilization.

For example, an anti-inflammatory substance can be added. Anti-inflammatory agents are a well known class of pharmaceutical agents which reduce inflammation by acting on body mechanisms (Stedman's Medical Dictionary 26 ed., Williams and Wilkins, (1995); Physicians Desk Reference 51 ed., Medical Economics, (1997)). Anti-inflammatory agents useful in the methods of the invention include, without limitation, non-steroidal anti-inflammatory agents (NSAIDS). NSAIDS typically inhibit the body's ability to synthesize prostaglandins. Prostaglandins are a family of hormone-like chemicals, some of which are made in response to cell injury. Specific NSAIDS approved for administration to humans include naproxen sodium, diclofenac, sulindac, oxaprozin, diflunisal, aspirin, piroxicam, indomethacin, etodolac, ibuprofen, fenoprofen, ketoprofen, mefenamic acid, nabumetone, tolmetin sodium, and ketorolac tromethamine. Other anti-inflammatory agents useful in the methods of the invention include salicylates, such as, for example, salicylic acid, acetyl salicylic acid, choline salicylate, magnesium salicylate, sodium salicylate, olsalazine, and salsa late. Still other anti-inflammatory agents useful in the methods of the invention include cyclooxygenase (COX) inhibitors. COX catalyzes the conversion of arachidonate to prostaglandin H2 (PGH2); a COX inhibitor inhibits this reaction. COX is also known as prostaglandin H synthase, or PGH synthase. Two Cox genes, Cox-1 and Cox-2 have been isolated in several species. COX-2 is tightly regulated in most tissues and usually only induced in abnormal conditions, such as inflammation, rheumatic and osteo-arthritis, kidney disease and osteoporosis. COX-1 is believed to be constitutively expressed so as to maintain platelet and kidney function and integral homeostasis. Typical COX inhibitors useful in the methods of the invention include etodolac, celebrex, meloxicam, piroxicam, nimesulide, nabumetone, and rofecoxib.

In some embodiments, anti-inflammatory agents that can be incorporated into a polymer matrix for administration in the methods of the invention include, 3-Amino-4-hydroxybutyric Acid, Aceclofenac, Acemetacin, Acetaminosalol, Alclofenac, Alminoprofen, α-Bisabolol, Paranyline, Amfenac, Bromfenac, Benoxaprofen, Benzpiperylon, Bermoprofen, Bromosaligenin, Bucloxic Acid, Bufexamac, Bumadizon, Butibufen, Carprofen, Cinmetacin, Clidanac, Clopirac, Diclofenac, Diclofenac Sodium, Diflunisal, Ditazol, Enfenamic Acid, c-Acetamidocaproic Acid Bendazac, Etodolac, Etofenamate, Felbinac, Fenbufen, Fenclozic Acid, Fendosal, Fenoprofen, Fentiazac, Fepradinol, Flufenamic Acid, Flunoxaprofen, Flurbiprofen, Gentisic Acid, Glucametacin, Glycol Salicylate, Ibufenac, Ibuprofen, Ibuproxam, Indomethacin, Indoprofen, Isofezolac, Isoxepac, Isoxicam, Ketoprofen, Ketorolac, Lomoxicam, Lonazola, Lonazolac, Loxoprofen, Meclofenamic Acid, Mefenamic Acid, Meloxicam, Mesalamine, Metiazinic Acid, Mofebutazone, Mofezolac, Naproxen, Niflumic Acid, Olsalazine, Oxaceprol, Oxametacine, Oxaprozin, Oxicams, Oxyphenbutazone, Paranyline, Parsalmide, Perisoxal, Phenyl Salicylate, Pirazolac, Piroxicam, Pirprofen, Pranoprofen, Proprionic Acids, Protizinic Acid, Salacetamide, Salicilic Acid, Salicylamide O-Acetic Acid, Salicylsulfuric Acid, Salsalate, Sulfasalazine, Sulindac, Suprofen, Suxibuzone, Talniflumate, Tenoxicam, Terofenamate, Tiaprofenic Acid, Tiaramide, Tinoridine, Tolfenamic Acid, Tolmetin, Tropesin, Xenbucin, Ximoprofen, Zaltoprofen, Zileuton and Zomepirac.

For any anti-inflammatory agent referred to herein by a trade name it is to be understood that either the trade name product or the active ingredient possessing anti-inflammatory activity from the product can be used.

The described agents useful in combination with LXA₄ and/or its analogs can be administered simultaneously, concurrently or sequentially as the oral compositions or indeed formulated together with the oral compositions.

### D. Resolvins

As used herein the term "resolvin" encompasses resolving, resolvin derivatives and analogs, as well as physiologically acceptable salts and prodrugs thereof. In certain embodiments, a single resolvin is administered to the subject. In other embodiments, two or more resolvins are administered to the subject. In such embodiments, administration of the resolvins may be simultaneous (i.e., administration at essentially the same time, e.g., in the form of a mixture of resolvins) or sequential (i.e., administration of the different resolvins at different times).

Resolvins are compounds generated from the interactions between a dietary omega-3-polyunsaturated fatty acid (PUFA) such as eicosapentaenoic acid (EPA) or docosahexaenoic acid (DHA), cyclooxygenase-II (COX-2) and an analgesic, such as aspirin ASA. It was recently demonstrated that ASA treatment of murine in vivo and human tissues in vitro carrying COX-2 initiates the production of novel 17R-hydroxy series docosanoids via previously undescribed pro-inflammatory responses (i.e., cytokine production, peritonitis). During stress, these cellular pathways utilize omega-3 fatty acids to biosynthesize endogenous compounds that serve in anti-inflammation signaling. These new di- and tri-hydroxy -containing compounds derived from omega-3 fatty acids were termed "resolvins", because they (a) are formed within the resolution phase of acute inflammatory response, at least in part, as cell-cell interactions products, (b) "stop" neutrophil entry to sites of inflammation, and (c) reduce exudates (C. N. Serhan et al., J. Exp. Med., 2002, 196: 1025-1037).

Compounds derived from eicosapentaenoic acid are designated as belonging to the E series, given their EPA precursor, and denoted as Resolvins of the E series (e.g., Resolvin E1 or RvE1). Compounds derived from docosahexaenoic acid are denoted as Resolvins of the D series (e.g., Resolvin D1 or RvD1).

Resolvins suitable for use in the methods of the present invention can be any member of the family of compounds known as resolvins, for example, as described in U.S. Pat. No. 6,949,664; U.S. Pat. Appln. Nos. 2005-0238589, 2005-0228047, 2005-0075398, 2004-0116408; and 2003-0191184; PCT application Nos. WO 2005/089744, WO 2005/013908, WO 2004/014835, WO 2003/084305, and WO 2003/053423; and European Pat. Appln. No. EP 1 537 067 Other suitable resolvins include those described, for example, in C. N. Serhan et al., J. Exp. Med., 2002, 196: 1025-1037; S. Hong et al., J. Biol. Chem., 2003, 278: 14677-14687; V. L. Marcheselli et al., J. Biol. Chem., 2003, 278: 43807-43817; C. N. Serhan and N. Chiang, Rheum. Dis. Clin. North Am., 2004, 30: 69-95; C. N. Serhan et al., Prostaglandins Other Lipid Mediat., 2004, 73: 155-172; C. N. Serhan et al., Histochem. Cell Biol., 2004, 122: 305-321; C. N. Serhan et al., Lipid, 2004, 39: 1125-1132; C. N. Serhan, Pharmacol. Ther., 2005, 105: 7-21; C. N. Serhan, Curr. Opin. Clin. Nutr. Metab. Care, 2005, 8: 115-121; G. L. Bannenberg et al., J. Immunol., 2005, 174: 4345-4355; U. N. Das, Med. Sci. Monit., 2005, 11: RA233-237; and U. N. Das, J. Assoc. Physicians India, 2005, 53: 623-527.

In certain embodiments, Resolvin E1 is used for enhancing or promoting plaque stabilization. Resolvin E1 belongs to an array of natural bioactive lipids that are generated in vivo from omega-3 polyunsaturated fatty acids by aspirin modified COX-2 (C. N. Serhan et al, J. Exp. Med., 2000, 192: 1197; C. N. Serhan et al., J. Exp. Med., 2002, 196: 1025). The Examples section below describes experiments in which Resolvin E1 is used.

Resolvins used in the methods and compositions of the present invention may be prepared in vivo or in vitro and then substantially purified and isolated by techniques known in the art (see, for example, U.S. Pat. No. 6,670,396). Without limitation, the purity of the compounds is generally at least about 90%, preferably at least about 95%, and most preferably at least about 99%. Certain Resolvins used in the inventive methods may be prepared by chemically modifying one or more purified compounds. For example, a purified compound may be chemically modified into a pharmaceutically acceptable salt or prodrug. Additionally or alternatively, one or more hydroxy, thiol or amino groups of the molecule may be protected using methods well known in the art. Resolvins can also be manufactured independently using conventional synthetic methods.

### E. Methods

The methods described herein relate, at least in part, to the treatment or prevention of arterial conditions that would benefit from increased plaque stabilization including, for example, atherosclerosis or diseases associated with atheroscleroris. It has been described herein that lipoxin A₄ (LXA₄), Resolvin E1, or analogs thereof can be used to increase plaque stabilization. Thus, lipoxin A₄ (LXA₄), Resolvin E1, or analogs thereof can be used in methods to increase plaque stabilization and thereby treat or prevent arterial conditions that would benefit from increased plaque stabilization, such as atherosclerosis or diseases associated with atherosclerosis. Such methods involve administering an LXA₄ and/or an LXA₄ analog to a subject in need of increased plaque stabilization.

As used herein, the term "agent" and "therapeutic agent" is defined broadly as anything that cells, tissues, or other surfaces of a subject having an arterial disorder that would benefit from increased plaque stabilization and may be exposed to in a therapeutic protocol.

The term "administering" is intended to include routes of administration which allow the agent to perform its intended function. Oral administration routes are preferred and, in some embodiments, the oral administration is targeted for application to the oral cavity, such as by applying the compositions and active ingredients contained therein to surfaces of the oral cavity, including but not limited to salivary glands, saliva, gingiva, tongue, cheek tissue, and the like. The orally administered agents may be administered alone, or in conjunction with a pharmaceutically acceptable carrier and/or other active ingredients, to prevent or treat an arterial disorder that would benefit from increased plaque stabilization agents, before, after, or simultaneously with the oral composition.

The term "effective amount" of an agent that treats or prevents an arterial disorder that would benefit from increased plaque stabilization is that amount necessary or sufficient to ameliorate the symptoms of the arterial disorder. The effective amount can vary depending on such factors as the type of therapeutic agent(s) employed, the size of the subject, or the severity of the disorder.

By "treatment," "prevention," or "amelioration" of an arterial disorder that would benefit from increased plaque stabilization is meant delaying or preventing the onset of such a disease or disorder, reversing, alleviating, ameliorating, inhibiting, slowing down or stopping the progression, aggravation, deterioration or severity of a condition associated with such a disease or disorder. The term "treatment" is intended to encompass also prophylaxis, therapy and cure. The efficacy of a given treatment can be determined by the skilled clinician. However, a treatment is considered "effective treatment," as the term is used herein, if any one or all of the signs or symptoms of the arterial disorder that would benefit from increased plaque stabilization of interest are altered in a beneficial manner, other clinically accepted symptoms or markers of disease are improved, or even ameliorated, e.g., by at least 10%, 20%, 30%, 40%, 50%, 60%, 70%, 80%, 90%, 95% or more following treatment with the agent. Efficacy can also be measured by a failure of an individual to worsen as assessed by hospitalization or need for medical interventions (*i.e.,* progression of the disease is halted or at least slowed). Methods of measuring these indicators are known to those of skill in the art and/or described herein. Treatment includes any treatment of a disease in an individual or an animal (some non-limiting examples include a human, or a mammal) and includes: (1) inhibiting the disease, e.g., arresting plaque rupture; or (2) relieving the disease, e.g., increasing plaque stability; and (3) preventing or reducing the likelihood of the development of a complication from the arterial disorder that would benefit from increased plaque stabilization.

In practicing some embodiments of the present invention, a safe and effective amount of the compositions of the present invention may be topically applied to oral bone surfaces, mucosal tissue of the oral cavity, to the gingival tissue of the oral cavity, to the tongue, to the salivary glands, to the saliva, and/or to the soft tissues, epithelium, and connective tissues, such as collagen and blood vessels for the treatment or prevention of the above mentioned diseases or conditions of the arteries, preferably for at least about from 0.1 to about 10 minutes, more preferably from 0.5 to 1 minute in several conventional ways. For example, the gingival or mucosal tissue may be rinsed with oral solution compositions (*e.g.,* mouth rinse, mouth spray) described herein. Other examples include applying a non-abrasive gel or paste formulation of the oral compositions describe herein directly to the gingival/mucosal tissue, salivary glands, saliva, tongue, or to the teeth with or without an oral care appliance. Chewing gum oral formulations can be chewed and lozenges or tablets can be sucked. The administration methods can be reapplied or repeated from 1 to about 5, preferably from 1 to 3 times per day. Alternatively or in addition, oral delivery can be performed at a frequency of once to several times per day for 1, 2, 3, 4, 5, 6, or 7 days; or 1, 2, 3, or 4 weeks; or 1, 2, 3, 4, 5, 6, 7, 8, 9, 10, 11, or 12 months or longer (or any range in between). Typically, the effective amount of the composition is from about 0.5 to about 10 grams, preferably about 1 gram.

More generally, it will be appreciated that individual dosages may be varied depending upon the requirements of the subject in the judgment of the attending clinician, the severity of the condition being treated and the particular compound being employed. In determining the therapeutically effective amount or dose, a number of additional factors may be considered by the attending clinician, including, : the pharmacodynamic characteristics of the particular activate ingredients and its mode of administration; the desired time course of treatment; the species of mammal; its size, age, and general health; the specific disease involved; the degree of or involvement or the severity of the disease; the response of the individual subject; the particular compound administered; the mode of administration; the bioavailability characteristics of the preparation administered; the dose regimen selected; the kind of concurrent treatment; and other relevant circumstances.

Treatment can be initiated with smaller dosages which are less than the effective dose of the compound. Thereafter, in one embodiment, the dosage should be increased by small increments until the optimum effect under the circumstances is reached. For convenience, the total daily dosage may be divided and administered in portions during the day if desired.

The effectiveness of any composition described herein to treat an arterial disorder that would benefit from increased plaque stabilization can be monitored by comparing two or more samples obtained from a subject undergoing treatment. In general, it is preferable to obtain a first sample from the subject prior to beginning therapy and one or more samples during treatment. In such a use, a baseline of expression of cells from subjects with the arterial disorder prior to therapy is determined and then changes in the baseline state of expression of cells from subjects with the arterial disorder that would benefit from increased plaque stabilization is monitored during the course of therapy. Alternatively, two or more successive samples obtained during treatment can be used without the need of a pre-treatment baseline sample.

In some embodiments, assays may involve subject samples. The term "sample," "tissue sample," "subject sample," "subject cell or tissue sample" or "specimen" each refer to a collection of similar cells obtained from a tissue of a subject. The source of the tissue sample may be solid tissue as from a fresh, frozen and/or preserved organ, tissue sample, or biopsy. The tissue sample may contain compounds that are not naturally intermixed with the tissue in nature such as preservatives, anticoagulants, buffers, fixatives, nutrients, antibiotics or the like.

In some embodiments, the amount and/or activity measurement(s) of a desired analyte, such as a blood protein, gene expression level, arterial tissue and the like in a sample from a subject is compared to a predetermined control (standard) sample. The sample from the subject is typically from an arterial source, such as saliva, dental plaque, gingiva, etc. The control sample can be from the same patient or from a different subject. The control sample is typically a normal, non-diseased sample. However, in some embodiments, such as for progression of disease or for evaluating the efficacy of treatment, the control sample can be from the subject at a different point in time or from a diseased tissue. The control sample can be a combination of samples from several different subjects. In some embodiments, the analyte amount and/or activity measurement(s) from a subject is compared to a pre-determined level. This pre-determined level is typically obtained from normal samples. As described herein, a "pre-determined" analyte amount and/or activity measurement(s) may be an analyte amount and/or activity measurement(s) used to, by way of example only, evaluate a subject that may be selected for treatment or evaluate a response to an plaque stabilization-promoting therapy. A pre-determined analyte amount and/or activity measurement(s) may be determined in populations of patients with or without the disorder. The pre-determined analyte amount and/or activity measurement(s) can be a single number, equally applicable to every patient, or the pre-determined analyte amount and/or activity measurement(s) can vary according to specific subpopulations of patients. Age, weight, height, and other factors of a subject may affect the pre-determined analyte amount and/or activity measurement(s) of the individual. Furthermore, the pre-determined analyte amount and/or activity can be determined for each subject individually, such as based on the average or median analyte level in a relevant cohort (e.g., similarly aged subjects or subjects having similar arterial disorder metrics). The pre-determined analyte amount and/or activity measurement(s) can be any suitable standard. For example, the pre-determined analyte amount and/or activity measurement(s) can be obtained from the same or a different human for whom a patient selection is being assessed. In one embodiment, the pre-determined analyte amount and/or activity measurement(s) can be obtained from a previous assessment of the same patient. In such a manner, the progress of the selection of the patient can be monitored over time. In addition, the control can be obtained from an assessment of another human or multiple humans, e.g., selected groups of humans, if the subject is a human. In such a manner, the extent of the selection of the human for whom selection is being assessed can be compared to suitable other humans, *e.g.,* other humans who are in a similar situation to the human of interest, such as those suffering from similar or the same condition(s) and/or of the same ethnic group or age.

In some embodiments of the present invention the change of analyte amount and/or activity measurement(s) from the pre-determined level is about 0.5 fold, about 1.0 fold, about 1.5 fold, about 2.0 fold, about 2.5 fold, about 3.0 fold, about 3.5 fold, about 4.0 fold, about 4.5 fold, or about 5.0 fold or greater. In some embodiments, the fold change is less than about 1, less than about 5, less than about 10, less than about 20, less than about 30, less than about 40, or less than about 50. In other embodiments, the fold change in analyte amount and/or activity measurement(s) compared to a predetermined level is more than about 1, more than about 5, more than about 10, more than about 20, more than about 30, more than about 40, or more than about 50.

The term "inhibit" includes the decrease, limitation, or blockage, of, for example a particular action, function, or interaction. In some embodiments, an arterial disorder that would benefit from increased plaque stabilization is "inhibited" if at least one symptom of the condition is alleviated, terminated, slowed, or prevented. In some embodiments, the term refers to a statistically significant decrease in a metric of interest indicative of an amelioration of an arterial disorder that would benefit from increased plaque stabilization. Such statistically significant decrease can be at least 10%, 20%, 30%, 40%, 50%, 60%, 70%, 80%, 90%, or more relative to a control. For example, a test compound administered and analyzed according to the methods described herein can comprise a *bona fide* increaser of plaque stabilization by increasing plaque stabilization by at least 10%, 20%, 30%, 40%, 50%, 60%, 70%, 80%, 90% or more relative to that of no agent administration or over a given amount of time.

The samples can be collected from individuals repeatedly over a longitudinal period of time (*e.g.,* once or more on the order of days, weeks, months, annually, biannually, etc.). Obtaining numerous samples from an individual over a period of time can be used to verify results from earlier detections and/or to identify an alteration in biological pattern as a result of, for example, disease progression, drug treatment, *etc.* For example, subject samples can be taken and monitored every month, every two months, or combinations of one, two, or three month intervals according to the invention. In addition, the analyte amount and/or activity measurements of the subject obtained over time can be conveniently compared with each other, as well as with those of normal controls during the monitoring period, thereby providing the subject's own values, as an internal, or personal, control for long-term monitoring.

### Exemplification

This invention is further illustrated by the following examples,

### Example 1: 9,12 benzo LXA₄ increases plaque stabilization in an atherosclerotic disease model

### A. Methods

New Zealand White (NZW) rabbits (n = 10) received cholesterol-enriched diet (1%) for 8 weeks to develop atherosclerosis. In addition, balloon injury was performed at 2 weeks to advance the atherosclerosis at the descending thoracic and abdominal aortic segments. The 6 weeks of cholesterol feeding was followed by a 4 week period of normal chow diet at the end of the experiment. Half of the rabbits were randomized in the experimental (9,12 benzo LXA4- treated) group and half in the control group.

Topical LXA4 (1mg/ml) was applied on both side of the mandible on the gingiva every-other-day for the duration of the experiment. In vivo MRI images were taken at 12 weeks at before and after triggering. After euthanasia, the aortas were dissected for ex-vivo MRI and histology.

In order to determine a disrupted plaque, MR images of plaque segments encompassing the region of interest (balloon injured) before and after the plaque triggering were compared and designated a plaque as disrupted if the post-trigger image showed a luminal thrombus. Of the 104 segments analyzed for the treatment group, only 2/104 showed disruption, which was only one site in one rabbit.

### C. Results

Of the 104 segments analyzed for the treatment group, only 2/104 showed disruption, which was only one site in one rabbit. For the control rabbits, a complete data set for 4 of the 5 rabbits was not obtained because of instrumental problems with the in vivo imaging. However, the one rabbit with complete in vivo data had disruptions in 6 of the 21 analyzed. 40/50 of the aortic segments have been scanned ex vivo at 11.7T.

## Claims

1. A composition comprising a therapeutically effective amount of 9,12 benzo-LXA₄ or Resolvin E1 for use in stabilizing an atherosclerotic plaque and/or reducing unstable/vulnerable plaque in a subject.

2. The composition for use of claim 1, wherein the subject is **characterized by** the presence of unstable/vulnerable atherosclerotic plaques.

3. The composition for use of claim 1 or 2, wherein the 9,12 benzo-LXA₄ or Resolvin E1 is formulated in a pharmaceutically acceptable carrier; optionally wherein the pharmaceutically acceptable carrier is selected from the group consisting of mouth rinses, chewing gum, food additives, lozenges, tablet, chewable capsule, intraoral delivery devices and encapsulated in resporbable carrier nanoparticles of biologic or synthetic origin.

4. The composition for use of claim 1, wherein the effective amount of the 9,12 benzo-LXA₄ or Resolvin E1 is an amount that increases plaque stabilization and/or reduces the number of unstable/vulnerable plaques.

5. The composition for use of claim 1, further comprising administering at least one additional agent selected from the group consisting of anti-inflammatory agents.

6. A composition comprising a therapeutically effective amount of 9,12 benzo-LXA₄ or Resolvin E1, for use in stabilizing an atherosclerotic plaque and/or reducing unstable/vulnerable plaque in a subject in need thereof comprising the steps of:
a) performing surgery upon at least one arterial lesion of the subject; and
b) administering the composition to the surgical wound at the time of surgery; to thereby stabilize an atherosclerotic plaque and/or reduce unstable/vunerable plaque in the subject.

7. The composition for use of claim 6, wherein the 9,12 benzo-LXA₄ or Resolvin E1 is formulated in a pharmaceutically acceptable carrier; optionally wherein the pharmaceutically acceptable carrier is selected from the group consisting of mouth rinses, chewing gum, food additives, lozenges, tablet, chewable capsule, intraoral delivery devices and encapsulated in resorbable carrier nanoparticles of biologic or synthetic origin.

8. The composition for use of claim 6, wherein the effective amount of the 9,12 benzo-LXA₄ or Resolvin E1 is an amount that increases plaque stabilization and/or reduces the number of unstable/vulnerable plaques.

9. The composition for use of claim 6, further comprising administering at least one additional agent selected from the group consisting of anti-inflammatory agents.

10. The composition for use of claim 6, further comprising the step of administering a therapeutically effective amount of 9,12 benzo-LXA₄ or Resolvin E1 to the at least one arterial lesion at least one time after surgery completion; or further comprises administering at least one additional agent selected from the group consisting of anti-inflammatory agents at least one time after surgery completion.

11. The composition for use of claim 1 or 6, wherein the administration is topical.

## Patentansprüche

1. Zusammensetzung, welche eine therapeutisch wirksame Menge von 9,12-Benzo-LXA₄ oder Resolvin E1 umfasst, zur Verwendung bei der Stabilisierung atherosklerotischer Plaques und/oder der Verringerung instabiler/gefährdeter Plaques in einer Person.

2. Zusammensetzung zur Verwendung nach Anspruch 1, worin die Person **gekennzeichnet ist, durch** das Vorhandensein instabiler/gefährdeter atherosklerotischer Plaques.

3. Zusammensetzung zur Verwendung nach Anspruch 1 oder 2, worin das 9,12-Benzo-LXA₄ oder Resolvin E1 in einem pharmazeutisch annehmbaren Träger formuliert ist; wahlweise worin der pharmazeutisch annehmbare Träger aus der Gruppe ausgewählt ist, bestehend aus Mundspülungen, Kaugummi, Nahrungsergänzungmitteln, Lutschtabletten, Tabletten, Kaukapseln, intraoralen Verabreichungseinrichtungen und in resorbierbarem Träger eingekapselten Nanopartikeln biologischen oder synthetischen Ursprungs.

4. Zusammensetzung zur Verwendung nach Anspruch 1, worin die wirksame Menge des 9,12-Benzo-LXA₄ oder Resolvin E1 eine Menge ist, die die Plaquestabilisierung erhöht und/oder die Anzahl der instabilen/gefährdeter Plaques reduziert.

5. Zusammensetzung zur Verwendung nach Anspruch 1, ferner umfassend die Verabreichung mindestens eines zusätzlichen Wirkstoffes, ausgewählt aus der Gruppe bestehend aus entzündungshemmenden Wirkstoffen.

6. Zusammensetzung, welche eine therapeutisch wirksame Menge von 9,12-Benzo-LXA₄ oder Resolvin E1 umfasst, zur Verwendung bei der Stabilisierung atherosklerotischer Plaques und/oder der Verringerung instabiler/gefährdeter Plaques bei einer Person, die dies benötigt, wobei die Verwendung die Schritte umfasst:
a) Durchführen einer Operation an mindestens einer arteriellen Läsion der Person; und
b) Verabreichen der Zusammensetzung an die chirurgische Wunde zum Zeitpunkt der Operation, um dadurch atherosklerotische Plaques zu stabilisieren und/oder instabile/gefährdete Plaques in dem Patienten zu reduzieren.

7. Zusammensetzung zur Verwendung nach Anspruch 6, worin das 9,12-Benzo-LXA₄ oder Resolvin E1 in einem pharmazeutisch annehmbaren Träger formuliert ist; wahlweise worin der pharmazeutisch annehmbare Träger ausgewählt ist aus der Gruppe bestehend aus Mundspülungen, Kaugummi, Nahrungsmittelzusätzen, Lutschtabletten, Tabletten, Kaukapseln, intraoralen Verabreichungsvorrichtungen und eingekapselten resorbierbaren Träger-Nanopartikeln biologischen oder synthetischen Ursprungs besteht.

8. Zusammensetzung zur Verwendung nach Anspruch 6, worin die wirksame Menge des 9,12-Benzo-LXA₄ oder Resolvin E1 eine Menge ist, die die Plaquestabilisierung erhöht und/oder die Anzahl der instabilen/ gefährdeten Plaques reduziert.

9. Zusammensetzung zur Verwendung nach Anspruch 6, wobei die Verwendung ferner die Verabreichung mindestens eines zusätzlichen Mittels umfasst, ausgewählt aus der Gruppe bestehend aus entzündungshemmenden Mitteln.

10. Zusammensetzung zur Verwendung nach Anspruch 6, wobei die Verwendung ferner den Schritt der Verabreichung einer therapeutisch wirksamen Menge von 9,12-Benzo-LXA₄ oder Resolvin E1 an die mindestens eine arterielle Läsion mindestens einmal nach Abschluss der Operation umfasst; oder ferner mindestens einmal nach Abschluss der Operation die Verabreichung mindestens eines zusätzlichen Mittels umfasst, ausgewählt aus der Gruppe bestehend aus entzündungshemmenden Mitteln.

11. Die Zusammensetzung zur Verwendung nach Anspruch 1 oder 6, wobei die Verabreichung topisch erfolgt.

## Revendications

1. Composition comprenant une quantité thérapeutiquement efficace de 9,12 benzo-LXA₄ ou Résolvine E1 pour une utilisation dans la stabilisation d'une plaque d'athérosclérose et/ou la réduction d'une plaque instable/vulnérable chez un sujet.

2. Composition pour une utilisation selon la revendication 1, dans laquelle le sujet est **caractérisé par** la présence de plaques instables/vulnérables d'athérosclérose.

3. Composition pour une utilisation selon la revendication 1 ou 2, dans laquelle le 9,12 benzo-LXA₄ ou la Résolvine E1 est formulé dans un vecteur pharmaceutiquement acceptable ; facultativement dans laquelle le vecteur pharmaceutiquement acceptable est choisi dans le groupe constitué par les bains de bouche, le chewing-gum, les additifs alimentaires, les pastilles, les comprimés, les capsules à mâcher, les dispositifs d'administration intra-buccale et les nanoparticules encapsulées dans un vecteur résorbable d'origine biologique ou synthétique.

4. Composition pour une utilisation selon la revendication 1, dans laquelle la quantité efficace du 9, 12 benzo-LXA₄ ou de la Résolvine E1 est une quantité qui augmente la stabilisation des plaques et/ou réduit le nombre de plaques instables/vulnérables.

5. Composition pour une utilisation selon la revendication 1, comprenant en outre l'administration d'au moins un agent supplémentaire choisi dans le groupe constitué par des agents anti-inflammatoires.

6. Composition comprenant une quantité thérapeutiquement efficace de 9,12 benzo-LXA₄ ou Résolvine E1, pour une utilisation dans la stabilisation d'une plaque d'athérosclérose et/ou la réduction d'une plaque instable/vulnérable chez un sujet qui en a besoin, comprenant les étapes de :
a) réalisation d'une chirurgie sur au moins une lésion artérielle du sujet ; et
b) administration de la composition à la plaie chirurgicale au moment de la chirurgie ;
pour ainsi stabiliser une plaque d'athérosclérose et/ou réduire la plaque instable/vulnérable chez le sujet.

7. Composition pour une utilisation selon la revendication 6, dans laquelle le 9,12 benzo-LXA₄ ou la Résolvine E1 est formulé dans un vecteur pharmaceutiquement acceptable ; facultativement dans laquelle le vecteur pharmaceutiquement acceptable est choisi dans le groupe constitué par les bains de bouche, le chewing-gum, les additifs alimentaires, les pastilles, les comprimés, les capsules à mâcher, les dispositifs d'administration intra-buccale et les nanoparticules encapsulées dans un vecteur résorbable d'origine biologique ou synthétique.

8. Composition pour une utilisation selon la revendication 6, dans laquelle la quantité efficace du 9, 12 benzo-LXA₄ ou de la Résolvine E1 est une quantité qui augmente la stabilisation des plaques et/ou réduit le nombre de plaques instables/vulnérables.

9. Composition pour une utilisation selon la revendication 6, comprenant en outre l'administration d'au moins un agent supplémentaire choisi dans le groupe constitué par des agents anti-inflammatoires.

10. Composition pour une utilisation selon la revendication 6, comprenant en outre l'étape d'administration d'une quantité thérapeutiquement efficace de 9,12 benzo-LXA₄ ou de Résolvine E1 à l'au moins une lésion artérielle, au moins une fois après l'achèvement de la chirurgie ; ou comprend en outre l'administration d'au moins un agent supplémentaire choisi dans le groupe constitué par des agents anti-inflammatoires, au moins une fois après l'achèvement de la chirurgie.

11. Composition pour une utilisation selon la revendication 1 ou 6, dans laquelle l'administration est topique.
